(19) Europäisches Patentamt
European Patent Office
Office européen des brevets

(11) Veröffentlichungsnummer: **0 150 030**
**A2**

(12) # EUROPÄISCHE PATENTANMELDUNG

(21) Anmeldenummer: 85100318.6

(22) Anmeldetag: 15.01.85

(51) Int. Cl.[4]: C 07 C 126/067

(30) Priorität: 19.01.84 DE 3401779

(43) Veröffentlichungstag der Anmeldung: 31.07.85 Patentblatt 85/31

(84) Benannte Vertragsstaaten: BE DE FR GB IT NL

(71) Anmelder: Linde Aktiengesellschaft
Abraham-Lincoln-Strasse 21
D-6200 Wiesbaden(DE)

(72) Erfinder: Ranke, Gerhard, Dipl. -Ing.
Feichtetstrasse 6
D-8134 Pöcking(DE)

(72) Erfinder: Schrader, Ulrich, Dr. Dipl.Ing.
Otto-Dischner-Weg 20
D-8000 München 60(DE)

(74) Vertreter: Schaefer, Gerhard, Dr.
Linde Aktiengesellschaft Zentrale Patentabteilung
D-8023 Höllriegelskreuth(DE)

(54) **Verfahren zur kombinierten Herstellung von Ammoniak und Harnstoff.**

(57) Es wird ein Verfahren zur kombinierten Herstellung von Ammoniak und Harnstoff durch Umsetzung von kohlenwasserstoffhaltigen Gasen mit Wasserdampf in einem Steamreformer (2) mit nachgeschaltetem Sekundärreformer (4) beschrieben, wonach das im gebildeten Gasgemisch vorhandene CO einer Konvertierung (7) zu $CO_2$ unterzogen wird, worauf das nunmehr im wesentlichen aus Wasserstoff, Stickstoff und Kohlendioxid bestehende Ammoniaksynthesegas einer Druckwäsche (9) zur Entfernung saurer Verunreinigungen, insbesondere $CO_2$, mit einem physikalisch wirkenden Lösungsmittel unterworfen wird, woraufhin der Druck über dem beladenen Lösungsmittel zur Ausgasung von koabsorbierten Inerten auf einen ersten Zwischendruck und anschließend zur Entfernung der Hauptmenge des absorbierten $CO_2$, das zur Harnstoffsynthese (20) herangezogen wird, auf einen zweiten Zwischendruck und schließlich zur Entfernung des Rest-$CO_2$ auf den Regenerierenddruck gesenkt wird (12), wonach das Lösungsmittel zur Druckwäsche zurückgeführt wird. Um das Kohlendioxid auf energie- und kostengünstige Weise aus dem Lösungsmittel zu entfernen und gleichzeitig Rein-$CO_2$ zu gewinnen, wird vorgeschlagen, das Lösungsmittel bei dem Regenerierenddruck mit Luft (15) als Strippgas zu behandeln (6) und das Strippgas zusammen mit dem ausgestrippten Rest-$CO_2$ in den Sekundärreformer (4) einzuspeisen (5).

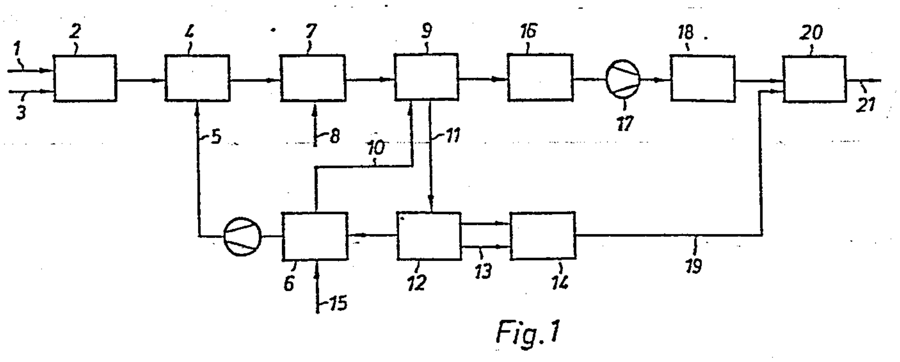


Fig. 1

EP 0 150 030 A2

Croydon Printing Company Ltd.

0150030

Verfahren zur kombinierten Herstellung von Ammoniak und Harnstoff

Die Erfindung betrifft ein Verfahren zur kombinierten Herstellung von Ammoniak und Harnstoff durch Umsetzung von kohlenwasserstoffhaltigen Gasen mit Wasserdampf in einem Steamreformer mit nachgeschaltetem Sekundärreformer, wonach das im gebildeten Gasgemisch vorhandene CO einer Konvertierung zu $CO_2$ unterzogen wird, worauf das nunmehr im wesentlichen aus Wasserstoff, Stickstoff und Kohlendioxid bestehende Ammoniaksynthesegas einer Druckwäsche zur Entfernung saurer Verunreinigungen, insbesondere $CO_2$, mit einem physikalisch wirkenden Lösungsmittel unterworfen wird, woraufhin der Druck über dem beladenen Lösungsmittel zur Ausgasung von koabsorbierten Inerten auf einen ersten Zwischendruck und anschließend zur Entfernung der Hauptmenge des absorbierten $CO_2$, das zur Harnstoffsynthese herangezogen wird, auf einen zweiten Zwischendruck und schließlich zur Entfernung des Rest-$CO_2$ auf den Regenerierenddruck gesenkt wird, wonach das Lösungsmittel zur Druckwäsche zurückgeführt wird.

Wie beispielsweise aus der DE-PS 27 21 462 bekannt ist, wird bei der Harnstoffsynthese von Ammoniak ausgegangen, das aus einem Gemisch aus Wasserstoff und Stickstoff

hergestellt wird.

Das im wesentlichen aus Wasserstoff, Stickstoff und Kohlendioxid bestehende Ammoniaksynthesegas wird zuerst einer Gaswäsche zur Entfernung von Kohlendioxid unterworfen. Aus dem selektiven Lösungsmittel dieser Waschkolonne wird in der anschließenden Regenerierkolonne Kohlendioxid drucklos wieder freigesetzt, welches dann im Rahmen der Harnstoffsynthese verwertet wird.

Für eine anschließende Harnstoffsynthese hat diese Verfahrensweise jedoch den Nachteil, daß dieses Kohlendioxid dann von Atmosphärendruck auf den Druck der Harnstoffsynthese von etwa 160 bar verdichtet werden muß, wozu ein sehr großer Energieaufwand erforderlich ist. Um diesen Nachteil zu vermeiden, wurde in einer älteren Patentanmeldung P 32 39 605.8 von der Anmelderin bereits vorgeschlagen, den Druck über dem beladenen Lösungsmittel nach der Teilentspannung auf einen Zwischenwert zu bringen und das Lösungsmittel zur Teilausgasung von $CO_2$ anzuwärmen. Das ausgasende $CO_2$ fällt somit unter Druck an und kann nach Abkühlung zur Harnstoffsynthese herangezogen werden. Das teilregenerierte Lösungsmittel wird einer letzten drucklosen Regenerierstufe unterworfen. Dort wird das Lösungsmittel zur Entfernung des Rest-$CO_2$ entweder mittels eines Reboilers weiter angewärmt oder durch Anwendung von Vakuum von dem Rest-$CO_2$ befreit.

Bei der erstgenannten Variante ergeben sich jedoch die Nachteile eines hohen Dampfbedarfes im Reboiler zur Restanwärmung des Lösungsmittels und zur Deckung der restlichen Entgasungswärme sowie von Fremdkälte zum Decken der Austauschverluste und zusätzlich eines Kältebedarfes zum Kompensieren der Pumpenerwärmung und Rohgasabkühlung. Hiermit verbunden ist ein zusätzlicher apparativer Aufwand, wie Wärmetauscher, Reboiler und Kondensator mit Ab-

0150030

scheider und Kondensatpumpe.

Die Verwendung von Vakuum erfordert ebenfalls eine zusätzliche Maschine mit relativ hohem spezifischem Energiebedarf. Die erreichbare Restbeladung ist durch den Saugdruck des Vakuumkompressors festgelegt. Somit kann das Rohgas in der Wäsche nicht so gut gereinigt werden. Die $CO_2$-Restbeladung kann gesenkt werden durch Anwärmung des Lösungsmittels mit Abwärme, doch dann treten ähnliche Nachteile wie bei der Warmregenierung auf.

Der vorliegenden Erfindung liegt daher die Aufgabe zugrunde, ein Verfahren der eingangs genannten Art so auszugestalten, daß auf energie- und kostengünstige Weise Kohlendioxid aus dem Lösungsmittel entfernt wird unter gleichzeitiger Gewinnung von Rein-$CO_2$.
Diese Aufgabe wird erfindungsgemäß dadurch gelöst, daß das Lösungsmittel bei dem Regenerierenddruck mit Luft als Strippgas behandelt wird und daß das Strippgas zusammen mit dem ausgestrippten Rest-$CO_2$ in den Sekundärreformer eingespeist wird.

Mit diesem Verfahren, das die Regeneriersäule durch eine Strippsäule ersetzt, kann ohne Dampfzuführung oder Verwendung von Vakuum eine Reinheit des Snythesegases von $CO_2$ erreicht werden, die eine anschließende Methanisierung des restlichen CO und $CO_2$ wirtschaftlich vertretbar macht. Gleichzeitig kann alles $CO_2$ als Produkt gewonnen werden, da das in den Sekundärreformer zurückgeführte $CO_2$ ohne Reaktion diesen verläßt und in einer Waschsäule erneut ausgewaschen wird. Somit wird zwar eine gewisse begrenzte Menge an $CO_2$ im Kreislauf geführt, doch ist diese Menge so gering, daß sie keinerlei negative Auswirkung auf die Effektivität des Verfahrens hat.

Das beladene Lösungsmittel wird stufenweise entspannt und anschließend mit Luft gestrippt. Somit ist für die Endregenerierung des Lösungsmittels keine Wärmezufuhr und auch kein Wärmetauscher für beladenes gegen regeneriertes Lösungsmittel erforderlich.

Die für die erfindungsgemäße Regenerierung des Lösungsmittels zur Verfügung stehende Luftmenge ist durch den Luftbedarf des Sekundärreformers festgelegt. Es ist daher in Weiterbildung des Erfindungsgedankens vorgesehen, den Regenerierenddruck so zu wählen, daß die zum $CO_2$-Abstrippen benötigte Luftmenge den Bedarf des Sekundärreformers deckt. Falls ein $N_2$-haltiges Erdgas im Steamreformer verarbeitet wird, sinkt der Luftbedarf für den Sekundärreformer ab, da der im Ammoniaksynthesegas benötigte Stickstoff teilweise über den Einsatzstoff zugeführt wird. Es empfiehlt sich daher, das Rohgas erfindungsgemäß vor der Wäsche zu verdichten und danach das $CO_2$ auszuwaschen. Da es sich bei der vorgesehenen Sauergaswäsche um eine physikalische Wäsche handelt, kann bei erhöhtem Druck mit einer kleineren Lösungsmittelmenge das $CO_2$ ausgewaschen werden. Gleichzeitig wird auch für die Regenerierung des beladenen Lösungsmittels weniger Luft benötigt.

Bei dieser Verfahrensweise wird zwar das im Rohgas enthaltene $CO_2$ ebenfalls verdichtet, doch kann bei der Entspannung des beladenen Lösungsmittels ein großer Teil des $CO_2$ unter höherem Druck wiedergewonnen werden, so daß der Gesamtenergiebedarf für Synthese und $CO_2$-Verdichtung nicht größer wird. Ein weiterer Vorteil des Verfahrens besteht darin, daß das bei der Zwischenentspannung anfallende $H_2$-reiche Gas mit dem Rohgasverdichter verdichtet werden kann, wodurch der Rückführverdichter eingespart wird.

Das erfindungsgemäße Verfahren kann bei allen physikalischen

Lösungsmitteln angewendet werden, wie beispielsweise bei Alkoholen, Ketonen, N-Methylpyrrolidon Polyäthylenglykoläthern, Dimethylformamid, Glykolen, Butyrolacton.

Im folgenden sei das erfindungsgemäße Verfahren anhand eines in zwei Figuren schematisch dargestellten Ausführungsbeispiels näher erläutert.

Es zeigen:

Figur 1 ein Blockdiagramm für das Gesamtverfahren und

Figur 2 ein Verfahrensschema für die Druckwäsche und anschließende Regenerierung.

Wie dem Blockdiagramm der Figur 1 zu entnehmen ist, wird über Leitung 1 ein kohlenwasserstoffhaltiges Gas, z.B. Erdgas oder Naphtha, einem Steamreformer 2 zugeführt. In diesem erfolgt eine katalytische Umsetzung des Erdgases oder auch Naphtha mit über Leitung 3 herangeführtem Wasserdampf im wesentlichen zu $H_2$+CO. Methan wird nur teilweise umgesetzt. Das reformierte Gas gelangt sodann in einen Sekundärreformer 4. Dort erfolgt die Umsetzung des restlichen Methan aus dem Gas nach dem Steamreformer mit Luft, die über Leitung 5 herangeführt wird. Die Luftmenge wird dabei so groß gewählt, daß vor der Ammoniaksynthese das geforderte Gemisch 3 $H_2$+$N_2$ erhalten wird. Zusammen mit dieser Luft wird auch das in einer Strippsäule 6 abgestrippte $CO_2$ den Sekundärreformer zugegeben und verläßt diesen wieder ohne Reaktion.

Das Gas aus dem Sekundärreformer wird sodann einer Konvertierung 7 zugeführt, in der das CO mit über Leitung 8 herangeführtem Wasserdampf zu $H_2$ und $CO_2$ entsprechend der Formel

$$CO + H_2O \longrightarrow H_2 + CO_2$$

umgesetzt wird. Der Restgehalt des konvertierten Gases an CO beträgt noch etwa 0,3%.

In einer der Konvertierung nachgeschalteten $CO_2$-Wäsche 9 wird das $CO_2$ mit einem physikalisch wirkenden Lösungsmittel, das über Leitung 10 aus der Strippsäule 6 herangeführt wird, bis auf einen Restgehalt von ca. 100 ppm $CO_2$ entfernt. Das beladene Lösungsmittel wird über Leitung 11 einer gegebenenfalls mehrstufigen Entspannung 12 unterzogen, bei der $CO_2$ ausgast, das über Leitungen 13 einem $CO_2$-Verdichter 14 zugeführt wird. Das teilregenerierte Lösungsmittel wird der Strippung 6 zugeführt, die in Gegenwart von über Leitung 15 zugeführter Luft durchgeführt wird. Das regenerierte Lösungsmittel gelangt sodann über Leitung 10 zurück zur Druckwäsche 9. Das ausgestrippte Rest-$CO_2$ wird zusammen mit Luft über Leitung 5 dem Sekundärreformer 4 zugeführt.

Das weitgehend von $CO_2$ befreite Gas aus der Druckwäsche 9 wird einer Methanisierung 16 zugeführt, in der eine katalytische Umsetzung von CO und Rest-$CO_2$ mit $H_2$ in Methan und Wasser stattfindet. Nach Verdichtung 17 des Gases findet in 18 die Ammoniaksynthese gemäß

$$3H_2 + N_2 \longrightarrow 2NH_3$$

statt. Schließlich wird das Ammoniak sowie verdichtetes $CO_2$ aus Leitung 19 einer Harnstoffsynthese 20 zugeführt, in der die Umsetzung von $NH_3$ und $CO_2$ zu Harnstoff erfolgt. Dieser wird über Leitung 21 abgezogen.

Die Druckwäsche und anschließende Regenerierung des beladenen Lösungsmittels ist in Figur 2 dargestellt. Das von der Konvertierung 7 kommende Gas in Leitung 22 wird in einer Waschsäule 23 mit regeneriertem Lösungsmittel, das der Säule im oberen Bereich über Leitung 24 aufgegeben

wird, gewaschen. Dabei wird $CO_2$ bis auf einen Restgehalt von ca. 100 ppm ausgewaschen. Die erreichbare Reinheit ist eine Funktion der Lösungsmittelmenge, der Temperatur und der Bodenzahl bzw. Schütthöhe.

Neben $CO_2$ werden entsprechend der jeweiligen Löslichkeit im Lösungsmittel auch andere Bestandteile des Rohgases gelöst, gleichzeitig wird auch Wasser teilweise ausgewaschen.

Das am Kopf der Waschsäule 23 abgezogene Gas wird über Leitung 25 der Methanisierung zugeführt.

Das am Sumpf der Waschsäule über Leitung 26 abgezogene beladene Lösungsmittel wird zunächst in einem Ventil 27 in einen Abscheider 28 auf einen Zwischendruck entspannt und die dabei freiwerdenden Gase, bevorzugt $H_2$ und $N_2$, über Leitung 29 über einen Rückführverdichter 30 ins Rohgas zurückgeführt. Dabei erfüllt dieser Rückführverdichter zwei Aufgaben: Erhöhung der $H_2$-Ausbeute und gleichzeitig Anhebung der $CO_2$-Konzentration im $CO_2$ zur Harnstoffsynthese.

Anschließend wird das Lösungsmittel in Leitung 31 schrittweise in Ventilen 32, 33 in Abscheider 34, 35 entspannt. Dabei wird jeweils $CO_2$ freigesetzt, welches über Leitungen 36, 37 mit einem $CO_2$-Verdichter 38 auf den Druck der Harnstoffsynthese verdichtet und dieser über Leitung 39 zugeführt wird. Die Anzahl der Entspannungsstufen und der Verdichterstufen wird sinnvollerweise aufeinander abgestimmt.

Nach Entspannung auf einen Druck von etwa 1,3 bar wird das Lösungsmittel, welches immer noch $CO_2$ enthält, über Leitung 40 einer Strippsäule 41 zugeführt und dort mit über Leitung 42 mit Verdichter 43 zugeführter Luft behandelt. Dabei wird die Luftmenge so gewählt, daß sie

anschließend im vorgeschaltetem Sekundärreformer zum Umsetzen des Methans und zum Einstellen des Synthesegasgemisches ausreicht. Mit dieser Luftmenge wird das im Lösungsmittel gelöste $CO_2$ abgetrieben und verläßt die Strippsäule 41 am Kopf über Leitung 44.

Das im Sumpf über Leitung 45 abgezogene Lösungsmittel wird auf den Druck der Waschsäule gepumpt, 46. In einem Kühler 47 wird die mit dem Rohgas und den Pumpen zugeführte Wärme abgeführt.

Zahlenbeispiel:

Auswaschung und Gewinnung von $CO_2$ aus Rohgas.
Mengenangaben entsprechend 1000 tato-$NH_3$-Anlage.
Verfahren: Das Rohgas wird vor der Wäsche auf 65 bar verdichtet.

| | | |
|---|---|---|
| Rohgas: | | 6781,5 kmol/h |
| Druck | | 65 bar |
| Zusammensetzung: | $H_2$ | 61,3 mol% |
| | $N_2$ | 20,05 mol% |
| | $CO+Ar+CH_4$ | 0,84 mol% |
| | $CO_2$ | 17,81 mol% |

| | | |
|---|---|---|
| Produktgas: | | 5548,5 kmol/h |
| Druck | | 63,5 bar |
| Zusammensetzung: | $H_2$ | 74,65 mol% |
| | $N_2$ | 24,34 mol% |
| | $CO+Ar+CH_4$ | 0,01 mol% |
| | $CO_2$ | 100 ppm |

<u>$CO_2$-Produkt</u> 1233,0 kmol/h

| | | |
|---|---|---|
| Druck | | 1,3 bzw. 3 bzw. 9 bar |
| Zusammensetzung: | $H_2$ | 1,1 mol% |
| | $N_2$ | 1,0 mol% |
| | $CO + CH_4$ | 0,2 mol% |
| | $CO_2$ | 97,7 mol% |

<u>Luft als Strippgas</u>: 1718 kmol/h

<u>Rückführung zum Sekundärreformer</u>: 1916 kmol/h

| | | |
|---|---|---|
| Druck vor Verdichter | | 1,3 bar |
| Zusammensetzung: | Luft | 89,5 mol% |
| | $CO_2$ | 10,5 mol% |

Lösungsmittelmenge: 800 m³/h eines Polyäthylenglykoläthers

0150030

## Patentansprüche

1. Verfahren zur kombinierten Herstellung von Ammoniak und Harnstoff durch Umsetzung von kohlenwasserstoffhaltigen Gasen mit Wasserdampf in einem Steamreformer mit nachgeschaltetem Sekundärreformer, wonach das im gebildeten Gasgemisch vorhandene CO einer Konvertierung zu $CO_2$ unterzogen wird, worauf das nunmehr im wesentlichen aus Wasserstoff, Stickstoff und Kohlendioxid bestehende Ammoniaksynthesegas einer Druckwäsche zur Entfernung saurer Verunreinigungen, insbesondere $CO_2$, mit einem physikalisch wirkenden Lösungsmittel unterworfen wird, woraufhin der Druck über dem beladenen Lösungsmittel zur Ausgasung von koabsorbierten Inerten auf einen ersten Zwischendruck und anschließend zur Entfernung der Hauptmenge des absorbierten $CO_2$, das zur Harnstoffsynthese herangezogen wird, auf einen zweiten Zwischendruck und schließlich zur Entfernung des Rest-$CO_2$ auf den Regenerierenddruck gesenkt wird, wonach das Lösungsmittel zur Druckwäsche zurückgeführt wird, dadurch gekennzeichnet, daß das Lösungsmittel bei dem Regenerierenddruck mit Luft als Strippgas behandelt wird und daß das Strippgas zusammen mit dem ausgestrippten Rest-$CO_2$

in den Sekundärreformer eingespeist wird.

2. Verfahren nach Anspruch 1, dadurch gekennzeichnet, daß der Regenerierenddruck so gewählt wird, daß die zum $CO_2$-Abstrippen benötigte Luftmenge den Bedarf des Sekundärreformers deckt.

3. Verfahren nach Anspruch 1 oder 2, dadurch gekennzeichnet, daß das Rohgas vor der Wäsche verdichtet wird.

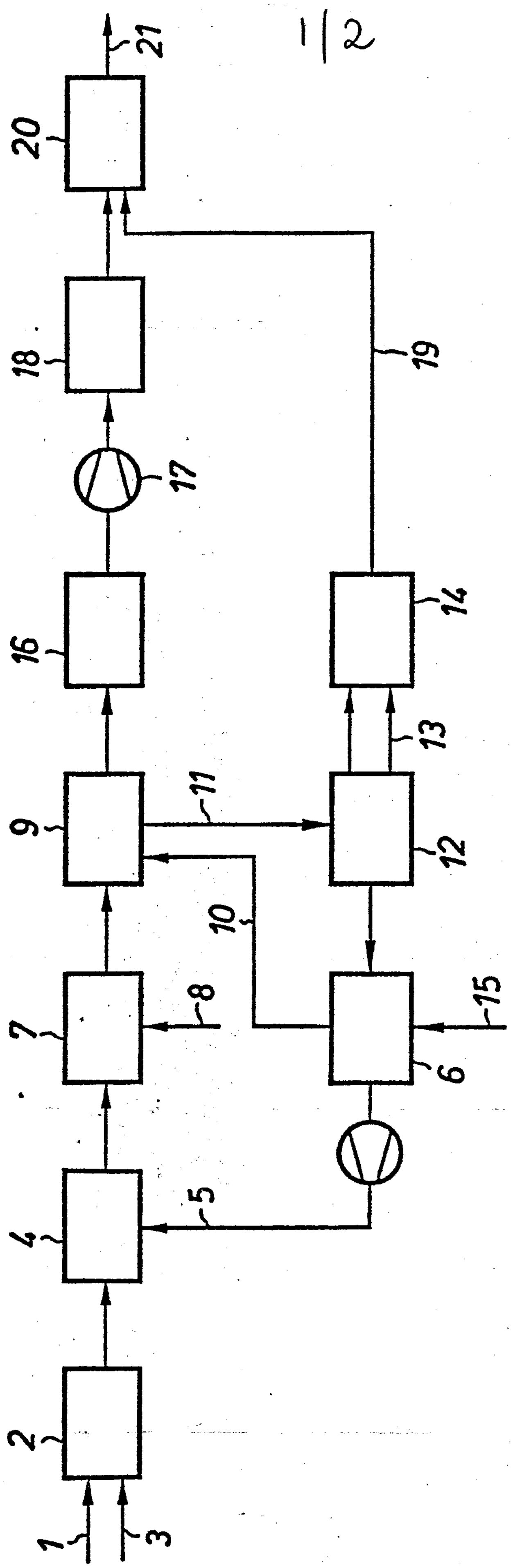


Fig.1

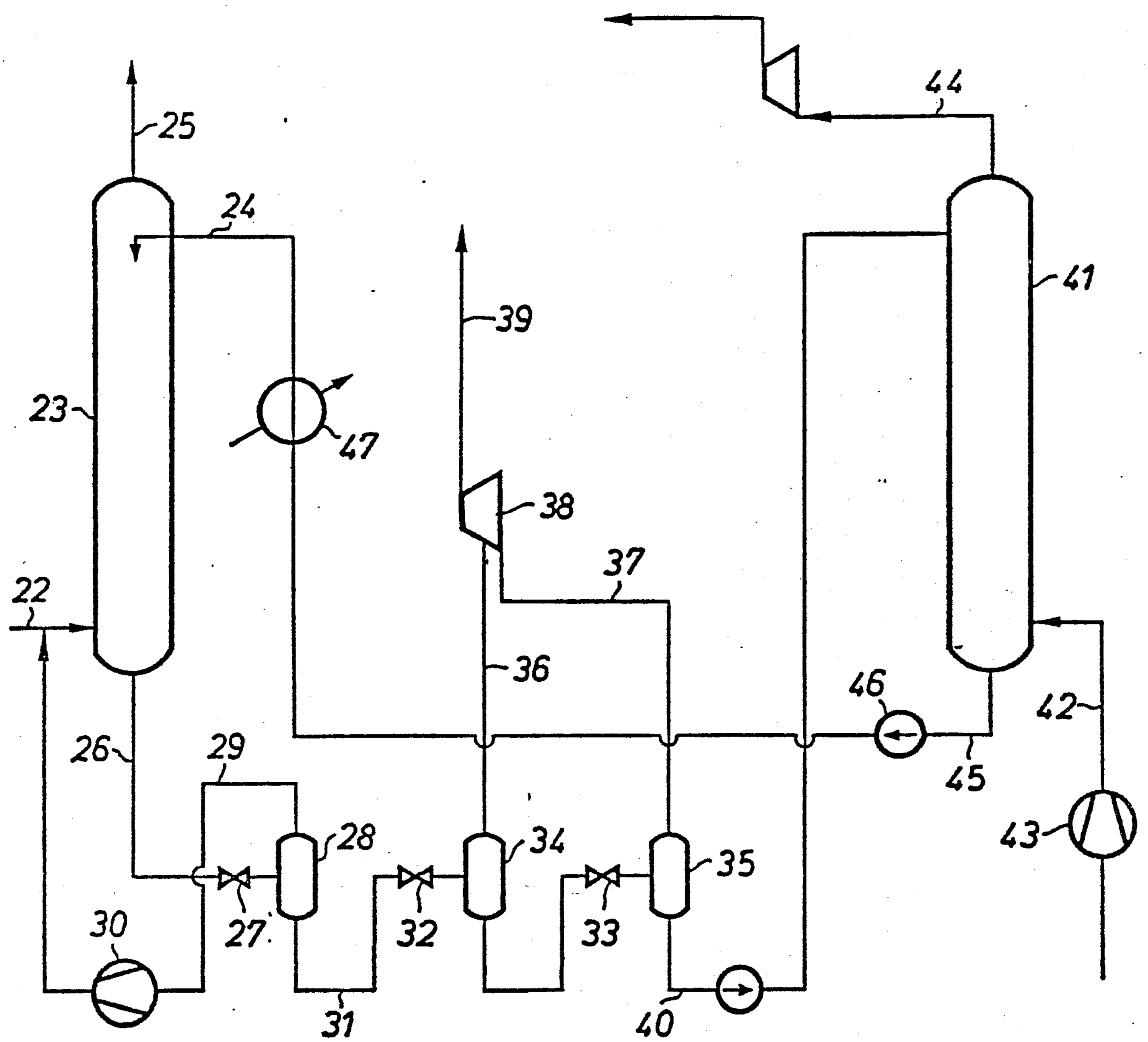

25
24
23
22
26
29
28
27
30
31
47
39
38
37
36
34
32
35
33
40
44
41
46
45
42
43


Fig. 2